(19) Europäisches Patentamt European Patent Office Office européen des brevets

(11) **EP 4 527 433 A1**

(12) **EUROPEAN PATENT APPLICATION**

(43) Date of publication:
26.03.2025 Bulletin 2025/13

(21) Application number: 23306569.7

(22) Date of filing: **21.09.2023**

(51) International Patent Classification (IPC):
*A61M 5/315* (2006.01)

(52) Cooperative Patent Classification (CPC):
A61M 5/31513; A61M 5/31515

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Becton Dickinson France
38800 Le Pont-de-Claix (FR)**

(72) Inventors:
• **EUVRARD, Nicolas**
London SW17 0JF (GB)
• **FLIPPE, Marc**
38640 CLAIX (FR)

(74) Representative: **Germain Maureau**
**12, rue Boileau**
**69006 Lyon (FR)**

(54) **SYRINGE PLUNGER ASSEMBLY WITH REDUCED-DIAMETER PLUNGER ROD HEAD**

(57) Provided herein is a syringe including a syringe barrel and a plunger assembly axially movable within a chamber of the syringe barrel. The plunger assembly includes a plunger rod having a plunger rod head at a distal end thereof and a stopper secured to the plunger rod head, with the plunger rod head including a head shaft and a head tip. The stopper includes a main body portion having proximal and distal ends and defining a cavity therein that mates with the plunger rod head, and a plurality of ribs extending around an outer circumference of the main body portion, with the plurality of ribs including at least a distal rib and a proximal rib. A ratio of an outer diameter of the head shaft, $OD_{head\_shaft}$, to an outer diameter of the stopper at the proximal rib, $OD_{rib\_prox}$, defined as $R = OD_{head\_shaft}/OD_{rib\_prox}$, is 0.4 or less.

FIG. 4

EP 4 527 433 A1

**Description**

**BACKGROUND OF THE INVENTION**

Field of the Invention

The present disclosure relates generally to syringes and, more particularly, to a plunger assembly of a syringe that includes a thin head plunger rod that mates with a stopper.

Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The head portion of the stopper may include a plurality of annular, outwardly protruding ribs formed on an external cylindrical wall thereof, with the ribs forming a seal with the syringe barrel to ensure the container closure integrity of the syringe. A series of two or three ribs, for example, may be spaced apart longitudinally along the stopper main body. The tail portion of the stopper may include a cavity formed therein configured to receive a distal head of the plunger rod, so as to engage the plunger rod with the stopper. According to some embodiments, each of the cavity and the plunger head may be configured to provide a threaded engagement therebetween to secure the plunger rod to the stopper.

In existing syringe designs, the stopper and the distal head of the plunger rod are designed to each have a "wide" design as compared to the overall width of the stopper. As one example, a stopper having an outer diameter of around 9.0 mm may include a cavity formed in the tail portion thereof having an inner diameter of 6.0 mm, with the plunger head having an outer diameter just slightly smaller than the inner diameter of the cavity, so as to fill a majority of the cavity when mated therewith. As another example, a stopper having an outer diameter of around 6.6 mm may include a cavity formed in the tail portion thereof having an inner diameter of 4.0 mm, with the plunger head again having an outer diameter just slightly smaller than the inner diameter of the cavity, so as to fill a majority of the cavity when mated therewith.

While this "wide" design of the stopper cavity and plunger head is effective in mating the plunger rod with the stopper, the design may negatively affect the syringe design and use thereof. That is, the wide design of the stopper plunger head may result in an increase in the radially outward force applied by the back stopper rib against the syringe barrel, due to the plunger head increasing the stiffness of this area of the stopper. The increased contact pressure applied by the back stopper rib thereby increases a gliding force of the stopper when the plunger assembly is pushed/advanced distally into-/through the syringe barrel. This increase in the gliding force may make it more difficult for an operator to advance the plunger assembly in a smooth and effortless manner, and may also negatively affect the seal quality between the stopper and the syringe barrel.

Accordingly, a need exists in the art for a plunger assembly design that limits a radial force applied by the back stopper rib against the syringe barrel with the plunger head inserted into the stopper cavity. The plunger rod assembly design would allow for a consistent and desirable gliding force to be maintained between the stopper and the syringe barrel, so as to enable a user-friendly operation of the syringe.

**SUMMARY OF THE INVENTION**

Provided herein is a syringe including a syringe barrel having a barrel proximal end and a barrel distal end and defining a chamber configured for containing a fluid therein, and a plunger assembly axially movable within the chamber of the syringe barrel between a retracted position and an advanced position. The plunger assembly includes a plunger rod having a plunger rod proximal end and a plunger rod distal end, with the plunger rod distal end comprising a plunger rod head including a head shaft and a head tip. The plunger assembly also includes a stopper secured to the plunger rod distal end. The stopper includes a main body portion defining a proximal end and a distal end, with the main body defining an opening at the proximal end providing access to an inner cavity of the main body portion that is configured to mate with the plunger rod head to secure the stopper to the plunger rod. The stopper also includes a plurality of ribs extending from an outer surface of the main body portion and around an outer circumference of the main body portion, with the plurality of ribs including at least a distal rib and a proximal rib. A ratio, R, of an outer diameter of the head shaft, $OD_{head\_shaft}$, to an outer diameter of the stopper at the proximal rib, $OD_{rib\_prox}$, defined as R = $OD_{head\_shaft}/OD_{rib\_prox}$, is 0.4 or less.

In certain configurations, the stopper comprises a threaded inner surface defining the cavity, and wherein the head tip comprises a flat head with two radially opposed wings configured to engage the threaded inner surface via a threading engagement.

In certain configurations, the stopper comprises a threaded inner surface defining the cavity, and wherein the head tip comprises a flat head with two radially opposed wings configured to engage the threaded inner surface via a threading engagement.

In certain configurations, the cavity comprises a main cylindrical cavity and a distal chamber separated by a bump, and wherein the head tip comprises a flat tip configured to engage the distal chamber and be retained therein by the bump.

In certain configurations, the stopper comprises a 1 mLl syringe stopper, with $OD_{rib\_prox}$= 6.9 mm, and wherein $OD_{head\_shaft} \leq 2.76$ mm, such that $R \leq 0.4$.

In certain configurations, the stopper comprises a 1-3 mL syringe stopper, with $OD_{rib\_prox}$= 9.2 mm, and wherein $OD_{head\_shaft} \leq 3.68$ mm, such that $R \leq 0.4$.

In certain configurations, the stopper comprises a 5 mL syringe stopper, with $OD_{rib\_prox}$= 12.5 mm, and wherein $OD_{head\_shaft} \leq 5$ mm, such that $R \leq 0.4$.

In certain configurations, the stopper comprises a 10 mL syringe stopper, with $OD_{rib\_prox}$= 15 mm, and wherein $OD_{head\_shaft} \leq 6$ mm, such that $R \leq 0.4$.

In certain configurations, the stopper comprises a 50 mL syringe stopper, with $OD_{rib\_prox}$= 27 mm, and wherein $OD_{head\_shaft} \leq 10.8$ mm, such that $R \leq 0.4$.

In certain configurations, a contact pressure applied by the first rib against the syringe barrel and a contact pressure applied by the second rib against the syringe barrel are equal.

In certain configurations, with the ratio, R, having a value of 0.4 or less, a radial outward stress applied by the stopper against the syringe barrel is reduced.

In certain configurations, the proximal rib is positioned on the main body portion so as to be axially aligned with a portion of the threaded inner cavity.

In certain configurations, the cavity is sized to receive a vent tube pushing rod therein, to enable placement of the stopper into the syringe barrel via a vent tube stoppering process.

In certain configurations, the stopper is formed of a first material and the plunger rod is formed of a second material, the second material having a greater stiffness than the first material.

In certain configurations, the stopper comprises a coating applied thereto, the coating comprising an inert material and/or a lubricant.

## BRIEF DESCRIPTION OF THE DRAWINGS

FIG. 1 is a perspective view of a syringe, with which embodiments of the disclosure may be implemented;

FIG. 2 is an exploded view of the syringe of FIG. 1;

FIG. 3 is a side view of the stopper included in the syringe of FIG. 1, according to a non-limiting embodiment described herein;

FIG. 4 is a side cross-sectional view of the mated stopper and plunger rod head of FIG. 1, taken along line 5-5, according to a non-limiting embodiment described herein;

FIG. 5 is a side cross-sectional view of the mated stopper and plunger rod head of FIG. 1, taken along line 5-5 according to another non-limiting embodiment described herein; and

FIG. 6 is a side cross-sectional view of the mated stopper and plunger rod head of FIG. 1, taken along line 5-5, according to another non-limiting embodiment described herein.

## DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

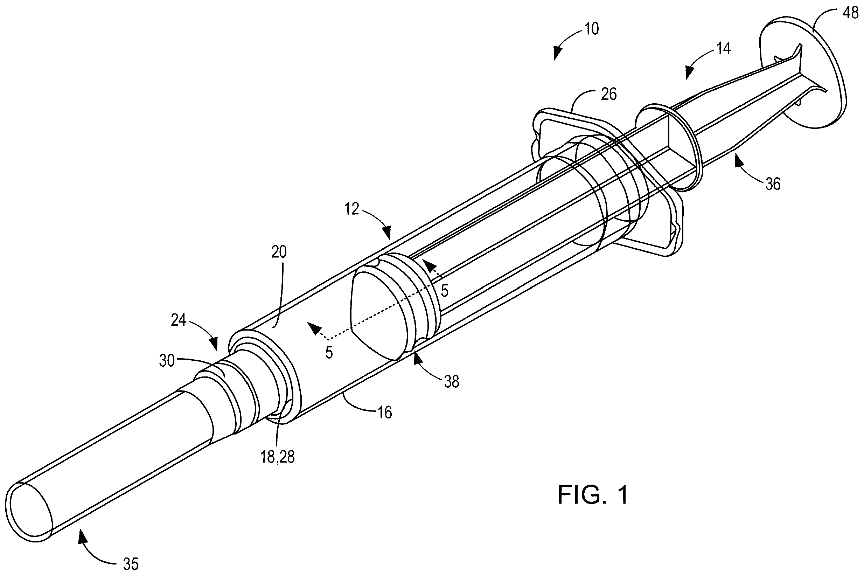

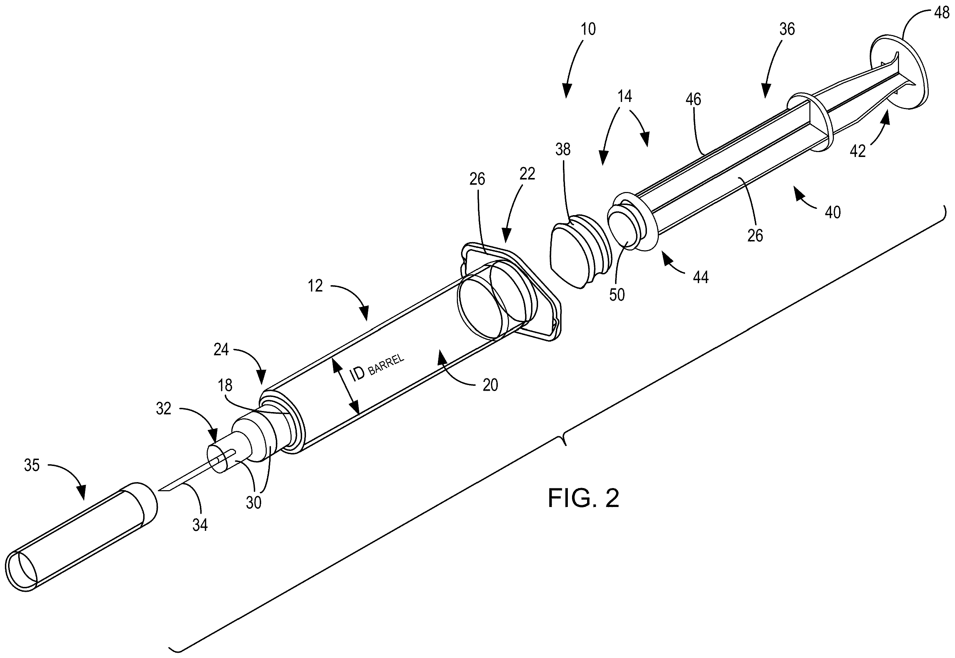

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. In some embodiments, a needle 34 is attached to the hub portion 30 within channel 32, such as by being glued or otherwise secured to the hub portion 30, although it is recognized that a luer connection could instead be provided at the distal end 24 of the syringe barrel 12. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. The plunger distal end 44 includes a plunger rod head 50 that extends out and is configured to mate with the stopper 38, with the stopper 38 secured to plunger rod head 50 at the plunger distal end 44 so as to be movable along with the plunger rod 36 within the chamber 20 of syringe barrel 12.

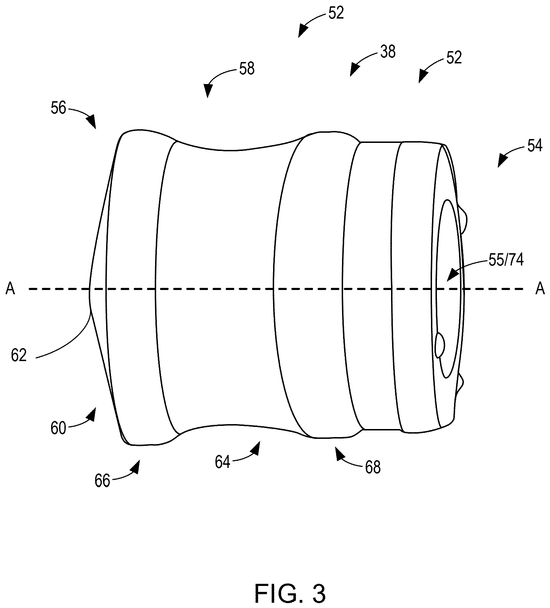

Referring now to FIGS. 3-6, and with continued reference to FIGS. 1 and 2, the structure of stopper 38 and plunger rod head 50 of plunger rod 36 are shown in greater detail, according to various aspects or embodiments of the disclosure. The stopper 38 is defined by a main body 52 that includes an open proximal end 54 (i.e., end 54 with opening 55) that engages with the plunger rod head 50 of plunger rod 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

An outer surface 64 of the main body 52 includes a plurality of ribs 66, 68 formed thereon that extend circumferentially around the entire outer surface 64. In the illustrated embodiment, the plurality of ribs 66, 68 includes a first (distal) rib 66 and a second (proximal) rib 68, although it is recognized that the stopper 38 could include a greater number of ribs, such as three or four ribs, according to other embodiments considered to be within the scope of the disclosure. The first and second ribs 66, 68 are spaced apart longitudinally, such that the first rib 66 is positioned toward the distal end 56 of the main body 52 and the second rib 68 is positioned toward the proximal end 54 of the main body 52, with an interib region present between the first and second ribs 66, 68.

The stopper 38 may be made from a material that is different from the material of the plunger rod 36 and that is capable of forming a tight seal with the syringe barrel 12 as it is advanced therethrough. According to embodiments, the stopper 38 may be formed of butyl rubber, styrene butadiene rubber, poly isoprene rubber, liquid silicone-rubber, or a thermoplastic elastomer (TPE), as non-limiting examples. In some embodiments, the stopper 38 may further include one or more outer coatings or layers 70 applied to all or portions of the outer surface 64 thereof, such as on a sealant or gliding surface 72 of first rib 66 and second rib 68 and/or on roof portion 60. The layer(s) 70 may be formed of an inert material that does not react with the composition contained within the syringe barrel 12 and/or may be configured to reduce friction between the stopper 38 and barrel 12 when the stopper 38 is moved within the syringe 10. According to non-limiting embodiments, the layer(s) 70 may be formed of a silicone oil, PTFE, ETFE, or liquid silicone rubber, as non-limiting examples.

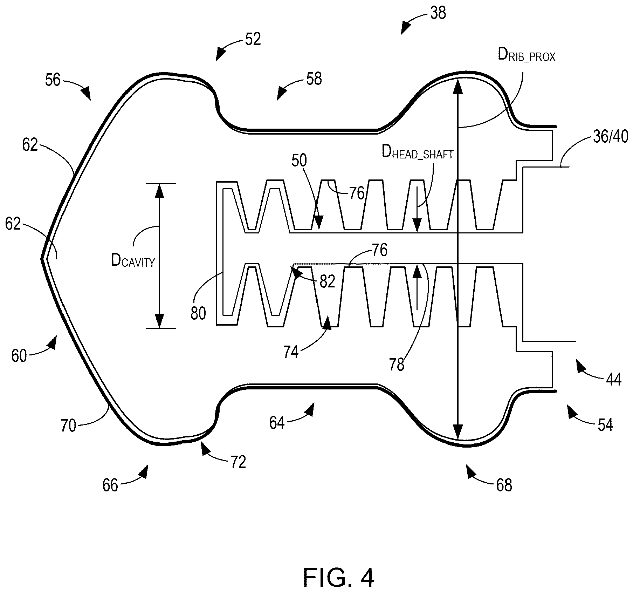

As shown in FIGS. 4-6, the main body 52 of the stopper 38 is partially hollow and is sized and configured to receive the plunger rod head 50 of plunger rod 36 therein. The main body 52 of the stopper 38 defines an inner cavity 74 that is generally defined between a majority of the open proximal end 54 and the closed distal end 56 of the main body 52. With the inner cavity 74 formed as such, the second rib 68 is thus positioned at a location that is axially aligned (along longitudinal axis A) with a portion of the inner cavity 74. The plunger rod head 50 may be characterized as including a head shaft 78 (hereafter "shaft 78") and a head tip 80 (hereafter "tip 80"), as shown best in FIGS. 4-6. The shaft 78 of plunger rod head 50 is joined to a distal end 44 of the main body 40 of plunger rod 36 and extends out distally therefrom, with the tip 80 formed at a distal end 82 of the shaft 78. According to aspects of the disclosure, the shaft 78 is configured to have a reduced diameter as compared to the main body 40 and tip 80, with the diameter of the shaft 78 being controlled/selected so as to provide a desired gliding force between the stopper 38 (i.e., second rib 68) and syringe barrel 12, as will explained in further detail below. The tip 80 is structured to provide for a secure engagement of the plunger rod head 50 within cavity 74, with the tip 80 having an increased size/diameter as compared to shaft 78 in order to mate with cavity 74.

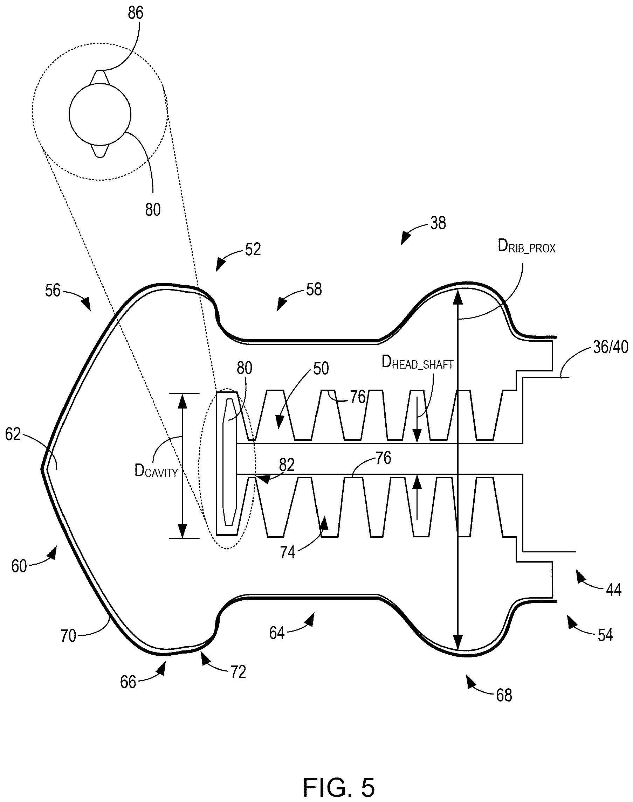

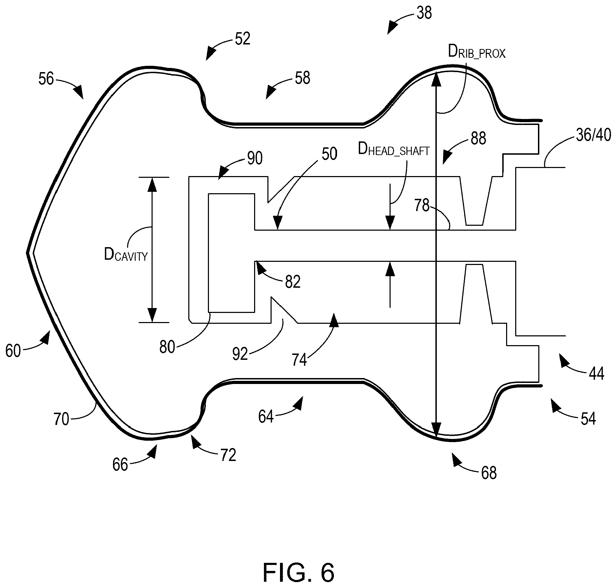

According to embodiments, the inner cavity 74 and the plunger rod head 50 may have any of a number of configurations that provide for mating and securing of the plunger rod 36 with the stopper 38. In one embodiment, and as shown in FIG. 4, the inner cavity 74 includes a threaded inner surface 76 that is configured to receive and mate with the plunger rod head 50 of plunger rod 36. The tip 80 of plunger rod head 50 may include a very short thread 84 thereon (e.g., a dual thread of half a turn), that matches the thread of threaded inner surface 76. In another embodiment, and as shown in FIG. 5, the inner cavity 74 may be configured to again include a threaded inner surface 76, but the tip 80 of plunger rod head 50 is configured to be flat with two radially opposed "wings" 86 that enable the plunger rod head 50 to be screwed into the threaded inner cavity 74. In still another embodiment, and as shown in FIG. 6, the inner cavity 74 may be configured to include a main cylindrical cavity 88 with a radially deeper chamber 90 at the end to accommodate a flat tip 80 of plunger rod head 50, with the main cavity 88 and deeper chamber 90 separated by a bump 92 in order to retain the tip 80 of plunger rod head 50 and prevent the plunger rod 36 from popping off from stopper 38.

It is recognized that the differing stiffness properties of the plunger rod head 50 (composed of plastic such as polypropylene, polycarbonate, etc.) and the stopper 38 (formed of rubber, TPE, etc.), can affect the performance of stopper 38 during use of the syringe 10. In particular, when the plunger rod head 50 is mated with the stopper 38, the radially outward-directed force or contact pressure applied by the stopper 38 against the syringe barrel 12 may differ at the locations of the first (distal) rib 66 and second (proximal) rib 68 due to the presence of the stiffer plunger rod head 50 within stopper cavity 74 - with the differing contact pressure impacting the gliding force when advancing the stopper 38 through the syringe barrel 12 during use of the syringe 10. The first rib 66 may apply a first radially outward-directed force against the syringe barrel 12 based on only the base stopper material (e.g., rubber or TPE) being present at this location - i.e., the first rib 66 is spaced axially from cavity 74, where plunger rod head 50 is positioned within stopper 38. Conversely, the second rib 68 may apply a second radially outward-directed force against the syringe barrel 12 (different from the first force) due to the second rib 68 being axially aligned with the cavity 74 and the stiffer plunger rod head 50 being positioned within the cavity 74 - with the presence of plunger rod head 50 increasing the (second) radially outward-directed force at the location of the second rib 68.

As best shown in FIGS. 4-6, the shaft 78 of plunger rod head 50 is specifically sized relative to the stopper 38 in order to reduce the impact/contribution that the plunger rod head 50 has on the radial stiffness of the stopper 38 when mated therewith. Specifically, the shaft 78 of plunger rod head 50 is configured to have an outer diameter, $OD_{head\_shaft}$, that is reduced in magnitude as compared to a typical plunger rod head diameter in known plunger assemblies. That is, existing plunger assembly designs include a "wide" plunger rod head configured to have an outer diameter that is similar in size to the cavity of the stopper, such that the plunger rod head substantially fills the cavity and increases the stiffness of the overall stopper/head combination - resulting in the stopper applying an increased radial force against the syringe barrel. This increased outwardly-directed radial force is applied by the rib(s) of the stopper 38 that are axially/longitudinally aligned with the cavity 74 and plunger rod head 50, such as at the second rib 68 of stopper 38, with the result being that the second rib 68 applies a greater radial force than the first rib 66.

According to aspects of the disclosure, for embodiments of the plunger assembly 14 where there is a radial interference between the stopper 38 and the shaft 78 of plunger rod head 50 (i.e., when the plunger assembly 14 is inserted into the syringe barrel 12, but not necessarily when not inserted in the syringe barrel 12) at a location axially aligned with the second (proximal) rib 68, the stiffness of the overall stopper/head combination at this axial location may be controlled via optimization of a ratio, R, of the outer diameter of the shaft 78 of the plunger rod head, $OD_{head\_shaft}$, to the outer diameter of the stopper 38 at the second (proximal) rib 68, $OD_{rib\_prox}$, with the ratio defined as:

$$R = \frac{OD\_head\_shaft}{OD\_rib\_prox}$$

As indicated above, by controlling the stiffness of the overall stopper/head combination at the location(s) of second (proximal) rib 68, the associated radial force applied by the second (proximal) rib 68 against the syringe barrel 12 may also be controlled. According to aspects of the disclosure, the ratio, R, of the outer diameter of the shaft 78 of plunger rod head 50, $OD_{head\_shaft}$, to the outer diameter of the stopper 38 at the second (proximal) rib 68, $OD_{rib\_prox}$, is controlled so that R is equal to or less than 0.40 (R ≤ 0.4) and preferably equal to or less than 0.3 (R ≤ 0.3). As it is recognized that the outer diameter of the stopper 38 at the second rib 68,

$OD_{rib\_prox}$, will be a set/standardized value that is determined by the volume of the syringe barrel (e.g., a 1-3 mL syringe, 5 mL syringe, 10 mL syringe, etc.), the outer diameter of the shaft 78 of plunger rod head 50, $OD_{head\_shaft}$, will be controlled/reduced in order to achieve a desired ratio, R.

In order to better illustrate aspects of the disclosure, provided here below are a number of non-limiting examples of exemplary outer diameters for each of the shaft 78 and second rib 68 for various well-known syringe sizes/volumes - with the diameter of the shaft selected to provide a ratio, R, at or below a desired value. While diameter values for a 1-3 mL syringe, a 5 mL syringe, a 10 mL syringe, and a 50 mL syringe are set forth below, it is recognized that aspects of the disclosure extend to all syringes volumes from 0.5 to 50 mL.

As a first example, when the syringe is a 1 mLl syringe, the stopper 38 has an outer diameter at the second rib 68 of $OD_{rib\_prox}$= 6.9 mm, while the shaft 78 of the plunger rod head 50 has an outer diameter of $OD_{head\_shaft} \leq 2.76$ mm -- such that R $\leq 0.4$.

As a second example, when the syringe is a 1-3 mL syringe, the stopper 38 has an outer diameter at the second rib 68 of $OD_{rib\_prox}$= 9.2 mm, while the shaft 78 of the plunger rod head 50 has an outer diameter of $OD_{head\_shaft} \leq 3.68$ mm -- such that R $\leq 0.4$.

As a third example, when the syringe is a 5 mL syringe, the stopper 38 has an outer diameter at the second rib 68 of $OD_{rib\_prox}$= 12.5 mm, while the shaft 78 of the plunger rod head 50 has an outer diameter of $OD_{head\_shaft} \leq 5$ mm -- such that R $\leq 0.4$.

As a fourth example, when the syringe is a 10 mL syringe, the stopper 38 has an outer diameter at the second rib 68 of $OD_{rib\_prox}$= 15 mm, while the shaft 78 of the plunger rod head 50 has an outer diameter of $OD_{head\_shaft} \leq 6$ mm -- such that R $\leq 0.4$.

As a fifth example, when the syringe is a 50 mL syringe, the stopper 38 has an outer diameter at the second rib 68 of $OD_{rib\_prox}$= 27 mm, while the shaft 78 of the plunger rod head 50 has an outer diameter of $OD_{head\_shaft} \leq 10.8$ mm -- such that R $\leq 0.4$.

With the ratio, R, minimized so as to be at or below a value of 0.4, as described above, there is a decrease in radial outward stress applied by the stopper 38 against the inner surface of the syringe barrel 12 (as compared to a typical ratio, R, of about 0.6 found with traditional stoppers. Assuming a polypropylene plunger rod 36 and a typical butyl rubber material for stopper 38, such decrease in the ratio, R, is thus expected to be about -30% to -35%. The decrease in radial stress provided by controlling of the ratio, R, should lead to a similar decrease in gliding force during advancement of the stopper 38 through the syringe barrel 12, assuming the same contact surface and friction coefficient (as it is understood that determining the gliding force also depends on the number of ribs, the interference level, the friction coefficient, and the contact surface).

Beneficially, embodiments of the invention thus are directed to a plunger assembly having a stopper and plunger rod head may be configured to achieve a desired contact pressure and gliding force between the stopper and syringe barrel. A shaft of the plunger rod head has a diameter that is specifically sized relative to the outer diameter of the stopper at a proximal rib thereof (at a region containing an inner cavity of the stopper) in order to reduce the impact/contribution that the plunger rod head has on the radial stiffness of the stopper when mated therewith - with a ratio of the shaft outer diameter to the proximal rib outer diameter being kept below an identified value to control the contact pressure applied by the proximal rib to the syringe barrel.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A syringe comprising:

a syringe barrel having a barrel proximal end and a barrel distal end and defining a chamber configured for containing a fluid therein; and
a plunger assembly axially movable within the chamber of the syringe barrel between a retracted position and an advanced position, the plunger assembly including:

a plunger rod having a plunger rod proximal end and a plunger rod distal end, the plunger rod distal end comprising a plunger rod head including a head shaft and a head tip; and
a stopper secured to the plunger rod distal end, the stopper further including:

a main body portion defining a proximal end and a distal end, with the main body defining an opening at the proximal end providing access to an inner cavity of the main body portion that is configured to mate with the plunger rod head to secure the stopper to the plunger rod; and
a plurality of ribs extending from an outer surface of the main body portion and around an outer circumference of the main body portion, the plurality of ribs including at least a distal rib and a proximal rib;

wherein a ratio, R, of an outer diameter of the head shaft, $OD_{head\_shaft}$, to an outer diameter of the stopper at the proximal rib, $OD_{rib\_prox}$, defined as $R = OD_{head\_shaft}/OD_{rib\_prox}$, is 0.4 or less.

2. The syringe of claim 1, wherein the stopper comprises a threaded inner surface defining the cavity, and wherein the head tip comprises a threaded member configured to engage the threaded inner surface via a threading engagement.

3. The syringe of claim 1, wherein the stopper comprises a threaded inner surface defining the cavity, and wherein the head tip comprises a flat head with two radially opposed wings configured to engage the threaded inner surface via a threading engagement.

4. The syringe of claim 1, wherein the cavity comprises a main cylindrical cavity and a distal chamber separated by a bump, and wherein the head tip comprises a flat tip configured to engage the distal chamber and be retained therein by the bump.

5. The syringe of any of claims 1-4, wherein the stopper comprises a 1 mLl syringe stopper, with $OD_{rib\_prox}=$ 6.9 mm, and wherein $OD_{head\_shaft} \leq 2.76$ mm, such that $R \leq 0.4$.

6. The syringe of any of claims 1-4, wherein the stopper comprises a 1-3 mL syringe stopper, with $OD_{rib\_prox}=$ 9.2 mm, and wherein $OD_{head\_shaft} \leq 3.68$ mm, such that $R \leq 0.4$.

7. The syringe of any of claims 1-4, wherein the stopper comprises a 5 mL syringe stopper, with $OD_{rib\_prox}=$ 12.5 mm, and wherein $OD_{head\_shaft} \leq 5$ mm, such that $R \leq 0.4$.

8. The syringe of any of claims 1-4, wherein the stopper comprises a 10 mL syringe stopper, with $OD_{rib\_prox}=$ 15 mm, and wherein $OD_{head\_shaft} \leq 6$ mm, such that $R \leq 0.4$.

9. The syringe of any of claims 1-4, wherein the stopper comprises a 50 mL syringe stopper, with $OD_{rib\_prox}=$ 27 mm, and wherein $OD_{head\_shaft} \leq 10.8$ mm, such that $R \leq 0.4$.

10. The syringe of any of claims 1-9, wherein a contact pressure applied by the first rib against the syringe barrel and a contact pressure applied by the second rib against the syringe barrel are equal.

11. The syringe of any of claims 1-10, wherein with the ratio, R, having a value of 0.4 or less, a radial outward stress applied by the stopper against the syringe barrel is reduced.

12. The syringe of any of claims 1-11, wherein the proximal rib is positioned on the main body portion so as to be axially aligned with a portion of the threaded inner cavity.

13. The syringe of any of claims 1-12, wherein the cavity is sized to receive a vent tube pushing rod therein, to enable placement of the stopper into the syringe barrel via a vent tube stoppering process.

14. The syringe of any of claims 1-13, wherein the stopper is formed of a first material and the plunger rod is formed of a second material, the second material having a greater stiffness than the first material.

15. The syringe of any of claims 1-14, wherein the stopper comprises a coating applied thereto, the coating comprising an inert material and/or a lubricant.

FIG. 1

FIG. 2

FIG. 3

FIG. 4

FIG. 5

FIG. 6

Europäisches
Patentamt

European
Patent Office

Office européen
des brevets

# EUROPEAN SEARCH REPORT

Application Number

EP 23 30 6569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/109250 A1 (GORE & ASS [US]) 27 May 2022 (2022-05-27) * paragraphs [0005], [0006], [0067], [0083], [0107]; figures 1,7,18,19,25A-D * ----- | 1-15 | INV. A61M5/315 |
| X | US 2016/243308 A1 (GIRAUD JEAN-PIERRE [US] ET AL) 25 August 2016 (2016-08-25) * paragraphs [0002], [0213], [0309]; figures 7,8,18,23-23A * ----- | 1-4,14, 15 | |

TECHNICAL FIELDS
SEARCHED        (IPC)

A61M

The present search report has been drawn up for all claims

| Place of search | Date of completion of the search | Examiner |
|---|---|---|
| Munich | 1 March 2024 | Diamantouros, S |

EPO FORM 1503 03.82 (P04C01)

**EP 4 527 433 A1**

## ANNEX TO THE EUROPEAN SEARCH REPORT
## ON EUROPEAN PATENT APPLICATION NO.

EP 23 30 6569

01-03-2024

| Patent document cited in search report | | | Publication date | Patent family member(s) | | | Publication date |
|---|---|---|---|---|---|---|---|
| WO 2022109250 | A1 | | 27-05-2022 | AU | 2021382676 | A1 | 06-07-2023 |
| | | | | CA | 3196910 | A1 | 27-05-2022 |
| | | | | CN | 116457043 | A | 18-07-2023 |
| | | | | EP | 4247459 | A1 | 27-09-2023 |
| | | | | JP | 2023550943 | A | 06-12-2023 |
| | | | | KR | 20230110316 | A | 21-07-2023 |
| | | | | US | 2024001036 | A1 | 04-01-2024 |
| | | | | WO | 2022109250 | A1 | 27-05-2022 |
| US 2016243308 | A1 | | 25-08-2016 | EP | 3055007 | A2 | 17-08-2016 |
| | | | | JP | 6673822 | B2 | 25-03-2020 |
| | | | | JP | 6991260 | B2 | 12-01-2022 |
| | | | | JP | 2016532469 | A | 20-10-2016 |
| | | | | JP | 2020096969 | A | 25-06-2020 |
| | | | | US | 2016243308 | A1 | 25-08-2016 |
| | | | | WO | 2015054282 | A2 | 16-04-2015 |